# EUROPÄISCHE PATENTSCHRIFT

(11) **EP 0 409 962 B1**
(45) Veröffentlichungstag und Bekanntmachung des Hinweises auf die Patenterteilung: **06.12.1995**
(21) Anmeldenummer: 90903364.9
(22) Anmeldetag: 09.02.1990
(51) Int. Cl.: A61K 38/55, C07K 5/02, C07K 7/02

(54) **MITTEL ZUR HEMMUNG VON HIV-PROTEASEN**
AGENT FOR INHIBITING HIV-PROTEASES
INHIBITEUR DES PROTEASES VIH

(30) Priorität: 10.02.1989 DE 3904040
(43) Veröffentlichungstag der Anmeldung: 30.01.1991
(73) Patentinhaber: SCHRAMM, Wolfgang, D-80336 München (DE); SCHRAMM, Hans J., D-82152 Planegg (DE)
(72) Erfinder: SCHRAMM, Wolfgang, D-80336 München (DE); SCHRAMM, Hans J., D-82152 Planegg (DE)
(74) Vertreter: Schüssler, Andrea, Dr.
(86) Internationale Anmeldenummer: EP9000219
(87) Internationale Veröffentlichungsnummer: WO9009191

(56) Entgegenhaltungen:
- EP-A- 0 077 028
- EP-A- 0 111 266
- EP-A- 0 352 000
- The Journal of Biological Chemistry, Band 263, Nr. 34, 5. Dezember 1988, Baltimore, MD, US; S. BILLICH et al.: "Synthetic Peptides as Substracted and Inhibitors of Human Immune Deficiency Virus-1 Protease", Seiten 17905-17908
- Biochemistry, Band 26, Nr. 18, 8. September 1987, Easton, PA, US; T.L. BLUNDELL et al.: "On the Rational Design of Renin Inhibitors: X-RAY Studies of Aspartic Proteinases Complexed with Transition-State Analogues", Seiten 5585-5590
- FEBS Letters, Band 247, Nr. 1, April 1989, Amsterdam, NL; I.V. PECHIK et al.: "Possible Role of Some Groups in the Structure and Function of HIV-1 Protease as Revealed by Molecular Modeling Studies", Seiten 118-122
- Kempf, D.J. et al.: J. ORG. CHEM., 1992, 57:5692-5700
- Toyoshima, T. et al.: J. CELL. BIOCHEM., 1992, Suppl.16E, Q558
- Kempf, D.J. et al.: J. MED. CHEM., 1993, 36:320-330
- Paessens et al.: IXth INT.CONF. AIDS, IVth STD World Congress, Berlin, 1993
- Tucker, T.J. et al.: J. MED. CHEM., 1992, 35:2525-2533
- Holmes, D.S. et al.: BIOORG. MED. CHEM. LETT., 1992, 3:503-508
- Yu, K-L et al: BIOORG. MED. CHEM. LETT., 1993, 3:535-539
- Peyman, A. et al.: TETRAHED. LETT., 1992, 33: 4549-4552

## Beschreibung

Die Erfindung betrifft ein Mittel mit einer zentralen Gruppe M und zwei Peptidseitenarmen zur Inhibierung der HIV-Proteinase bzw. Protease, in Form von strukturell symmetrisch oder fast oder teilweise symmetrisch gebauten Enzyminhibitoren.

Die spezifische Hemmung von Fremdenzymen (aus pathogenen Bakterien oder Viren) oder von körpereigenen Enzymen in pathologischen Zuständen ist ein wichtiges Anliegen der Medizin, da sie eine schonende Therapie von Erkrankungen erlaubt. Die Erfindung resultiert aus Versuchen, solche spezifischen Hemmstoffe für die Immunschwächekrankheit AIDS (Acquired Immune Deficiency Syndrome) zu finden. Sie erfolgten an der Proteinase, kurz auch "Protease" genannt, von HIV (Human Immunodeficiency Virus), einem spezifischen Enzym der AIDS verursachenden HI-Viren. Dieses Enzym ist für die Prozessierung der Vorläuferproteine verantwortlich. Es spaltet aus ihnen die fertigen Virusproteine heraus, aus denen dann das komplette Virus assembliert wird. Eine spezifische Hemmung der HIV-Protease sollte die Vermehrung der Viren unterbinden und die Symptome kurieren. Die Strategie der spezifischen Hemmung der Protease ist bei AIDS besonders bedeutungsvoll, da einmal immunologische Hemmansätze das Risiko in sich bergen, die restliche Immunabwehr des Körpers zu zerstören, und andererseits die Therapie unter Verwendung der bisher bekannten Hemmstoffe der Reversen Transkriptase von HIV (z.B. AZT, FLT, Suramin), einem anderen virusspezifischen Enzym, durch schwerste Nebenwirkungen beeinträchtigt ist. Auch die Therapie von AIDS mittels anderer Verbindungen (z.B. der polysulfatierten Polysaccharide) ist noch nicht überzeugend demonstriert worden oder auch mit schweren Nebenwirkungen belastet.

Es gibt zahlreiche Literatur über die HIV-Protease, wozu beispielsweise verwiesen sei auf
L.H. Pearl & W.R. Taylor, Nature (1987) 329, 351-354
I. Katoh et al., Nature (1987) 329, 654-656
C. Debouck et al., P.N.A.S. (1987) 84, 8903-8906
P.L. Darke et al., B.B.Res.Comm. (1988) 156, 297-303
S.F.J. Le Grice et al., EMBO J. (1988) 7, 2547-2553
M.C. Graves et al., P.N.A.S. (1988) 85, 2499-2453
M. Kotler et al., P.N.A.S. (1988) 85, 4185-4189
S. Billich et al., J.B.C. (1988) 263, 17905-17908
S. Seelmeier et al., P.N.A.S. (1988) 85, 6612-6616
E.P. Lillehoj et al., J. Virol. (1988) 62, 3053-3058
L.E. Henderson et al., J. Virol. (1988) 62, 2587-2595
H.-G. Kräusslich et al., J. Virol. (1988) 62, 4393-4397
M. Miller et al., J.Mol.Biol. (1988) 204, 211-212
Der Erfindung liegt die Aufgabe zugrunde, eine effektivere Beeinflussung von HIV-Proteasen (durch die zugehörigen Proteine) zu erreichen, z.B. um eine effektivere Therapie von AIDS und anderen Krankheiten, bei denen Enzyme involviert sind, zu erzielen. Eine hohe Spezifität und ein günstiger therapeutischer Index ist dabei von entscheidender Wichtigkeit.

Diese Aufgabe wird gelöst durch die HIV-Proteaseinhibitoren der Ansprüche 1 und 2, deren Molekül in bezug auf das zu hemmende Enzymmolekül strukturell von gleicher oder annähernd oder teilweise gleicher Symmetrie sind wie das zu hemmende Enzymmolekül.

Derartige Enzyminhibitoren sind also bezüglich ihrer Symmetrie maßgeschneidert im Hinblick auf die Symmetrie des zu hemmenden Enzyms, das für das Fortschreiten der Krankheit essentiell ist. Diese Inhibitoren werden in an sich bekannter Weise synthetisiert und dann in ebenfalls in der Arzneimitteltechnik üblichen Weise, z.B. i.v. oder oral verabreicht, wobei die Hemmung der Enzyme durch die Verbindungen eine brauchbare Therapie darstellt.

Es wurde festgestellt, daß strukturell symmetrisch gebaute (kurz "symmetrisch" genannte) Enzyminhibitoren besonders gut geeignet sind, um die Vermehrung von HI-Viren durch Hemmung der symmetrischen (aus zwei identischen Halbmolekülen bestehenden) viruskodierten Protease zu hemmen. Symmetrische oder teilweise symmetrische Enzyminhibitoren sind bekannt (z.B. für eine Reverse Transkriptase, über deren Struktur und Symmetrie jedoch noch nichts bekannt war), das vorliegende Wirkungsprinzip der Zueinanderpassung von Symmetrie des Enzyms und Symmetrie des Enzymhemmers jedoch nicht. Symmetrische Inhibitoren auf Peptidbasis sind, soweit bekannt, bisher nicht beschrieben worden und konnten auch nicht erwartet werden, da die natürlichen Substrate von Enzymen, auch von symmetrisch gebauten, nie symmetrisch sind. Der Erfindung liegt die Erkenntnis zugrunde, daß bei solchen Reaktionen (Bindung von unsymmetrischen Substraten an symmetrische Enzyme, bzw. Hemmung von symmetrisch gebauten Enzymen durch unsymmetrische Peptidinhibitoren) entweder nur eine - gut passende - Hälfte des Peptids für die Bindung verantwortlich ist und die andere Hälfte nur eine Hilfsfunktion besitzt oder daß beide Seiten nicht optimal passen, aber zusammen eine für die Hemmung ausreichende Affinität ergeben. Gut passende symmetrische Peptide und Peptidderivate (oder andere symmetrische organisch-chemische Verbindungen) sollten hingegen bei symmetrischen Enzymen allgemein eine stärkere Bindung (und gegebenenfalls Hemmung) vermitteln können als unsymmetrische Peptide.

Es wurde ferner erkannt, daß aus Untereinheiten bestehende Enzymkomplexe - symmetrische wie unsymmetrische - gehemmt werden können, wenn der Zusammenhalt der einzelnen Untereinheiten durch geeignete Verbindungen gestört wird, so daß entweder eine vollständige oder teilweise Zerlegung oder eine Labilisierung der Komplexe erfolgt, bzw. die Bildung der Komplexe oder der für die enzymatische Reaktion richtigen räumliche Struktur oder Konformation vollständig oder teilweise verhindert wird. Dies kann dadurch erreicht werden, daß Peptide oder peptidähnliche Verbindungen oder andere organisch-chemische Verbindungen angeboten werden, die Aminosäuresequenzen enthalten, oder von solchen abgeleitet sind oder mit solchen verwandt sind, die in den nativen Enzymen vorkommen und für den Zusammenhalt der für die Funktion nötigen Tertiär- und Quartärstruktur verantwortlich sind.

Dies gilt auch und vor allem in Hinsicht auf das aktive Zentrum der Enzymkomplexe, wo relativ kleine Störungen der Struktur bereits zur Inaktivierung des Enzyms führen können. Peptide mit Sequenzen der das aktive Zentrum bildenden oder dieses stabilisierenden Peptidketten (oder Verbindungen mit ähnlicher Struktur) sind daher besonders geeignet, die Aktivität des Enzyms zu hemmen, indem sie die Struktur stören oder die Bildung der korrekten räumlichen Enzymstruktur verhindern. Die hier einsetzbaren Verbindungen müssen selbst nicht symmetrisch sein, die Wirkung ist aber bei symmetrischen Enzymen besonders effektiv, da bei diesen mehrere identische Bindungsstellen vorhanden sind und die Wirkung sich daher je nach Anzahl der Untereinheiten im Komplex vervielfacht. Dieses Prinzip gilt auch für nichtsymmetrische, aber aus Untereinheiten zusammengesetzte Proteine.

Die Vorteile sind eine sehr spezifische Hemmung der Proteine (Enzyme bzw. Proteasen), da es die genauen strukturellen Eigenheiten der Zielproteine berücksichtigt und die Eigenschaft der Symmetrie für eine verstärkte Bindung der Hemmstoffe aufgrund der mindestens verdoppelten Bindungsfläche ausnützt.

Bei AIDS - wie auch bei anderen Krankheiten - sollte die hohe Spezifität und Bindungskraft der Inhibitoren eine relativ schonende Behandlung erlauben. Dies ist bei AIDS besonders wichtig, da dieses Krankheit eine sehr schonende Behandlung benötigt, weil AIDS das Immunsystem schädigt und daher die Anfälligkeit des Körpers gegen Krankheiten aller Art drastisch zunimmt. Zum anderen wird gerade bei AIDS, das nicht kausal kuriert werden kann, da die Virus-Nukleinsäure in das Genom eingebaut wird, eine lebenslängliche Therapie und damit eine sehr schonende und spezifische Behandlung nötig sein.

Ein solches Mittel zeichnet sich insbesondere dadurch aus, daß es eine zentrale organisch-chemische Gruppe aufweist, die der Einfachheit halber M genannt werden soll, an die als Seitenketten Reste X, Y, Z, U, R gebunden sind, welche Aminosäuren oder Aminosäurederivate sein können, die jeweils gleich oder annähernd gleich und in bezug auf die Gruppe M symmetrisch oder annähernd symmetrisch sind, so daß sich insgesamt eine symmetrische oder annähernd oder teilweise symmetrische Verbindung ergibt. Der Begriff "Symmetrie" ist hier im üblichen Sinn der Stereochemie zu verstehen, bezieht sich bei Proteinen also immer auf eine Drehachse.

Somit können durch solche Hemmstoffe HIV-Proteasen gehemmt werden, wenn sie zumindest bezüglich des hemmbaren Molekülteils eine lokale Symmetrie besitzen.

Die symmetrischen Inhibitoren für die symmetrischen Proteine haben dieselbe Symmetrie wie die zu hemmenden Proteine und bestehen wie erwähnt aus einer zentralen Gruppe M und Peptid-Seitenarmen, welche der Einfachheit halber im vorliegenden Fall nur mit X bezeichnet werden sollen, so daß sich eine Formel nach dem Schema

M(X)ₙ

ergibt, wobei n die Zähligkeit der Symmetrie des Proteins ist, z.B. "2" bei Proteinen mit 2-zähliger Achse, Symmetrie C₂).

Wie erwähnt, können die Seitenarme bestehen aus Aminosäuren, oder Derivaten davon. Peptide haben unter anderem den Vorteil, durch leichte, zum Teil automatisierte Synthese zugänglich zu sein. Bevorzugt werden vor allem kurze Peptide meist mit 2 bis 4 Aminosäuren pro Seitenarm verwendet.

Die beiden Arme müssen zueinander symmetrisch oder annähernd oder teilweise symmetrisch sein, in dem Sinne, daß die Symmetrie des zu hemmenden Proteins, z.B. eine 2-Zähligkeit, sich in dem Inhibitor wiederfindet. Im Beispiel der Dyade muß also beim Inhibitor durch Drehen um die zweizählige Achse ein Seitenarm in den anderen übergeführt werden können.

Dies kann z.B. auf folgende Weise erreicht werden:
a) Wenn die Laufrichtung der Peptidketten in den beiden Hälften verschieden ist, dergestalt, daß in einer Hälfte der NH-CO-Vektor zum Zentrum weist, in der anderen Hälfte vom Zentrum weg, dann muß in einer Hälfte durch Verwendung von Aminosäuren entgegengesetzter Chiralität (D statt L, bzw. umgekehrt) ein Ausgleich geschaffen werden. Der so entstandene Inhibitor ist dann bezüglich der Seitenketten noch annähernd symmetrisch, bezüglich der Peptidbindungen allerdings nicht. Dies genügt aber in aller Regel, daß der Hemmer für das zu hemmende Enzym noch hinreichend symmetrisch ist.
b) Wenn nicht alle Aminosäuren oder sonstige Reste des Inhibitors symmetrisch sind, sondern z.B. ein Tyrosin auf einer Seite durch ein Phenylalanin ergänzt wird, die restlichen Aminosäuren aber gleich und komplementär sind, ist immer noch mit einer hohen Hemmaktivität zu rechnen. Solche Hemmstoffe können sogar bezüglich der Löslichkeit, Membrangängigkeit und dergleichen günstiger sein alls streng symmetrische Verbindungen. Für die Abweichung sind strukturelle und physikalisch-chemische Parameter wie Größe, Ladung, Hydrophilizität und dergleichen, maßgebend. So ist der Inhibitor
   Phe-Thr-Ile-M-Leu-Ser-Tyr
   bezüglich der genannten Eigenschaften "symmetrischer" als
   Ala-Arg-Gly-M-Gly-Asp-Ala (ungleiche Ladung, Arg/Asp))
   oder
   Gly-Gly-Try-M-Gly-Gly-Gly (ungleiche Größe, Try/Gly), da der Unterschied, z.B. zwischen Thr einerseits und Ser andererseits geringer ist als zwischen Arg und Asp oder zwischen Try und Gly.

Kleine Abweichungen von der Symmetrie können daher prinzipiell geradezu von Vorteil sein. Es muß nur der Gewinn an Affinität durch die optimale Strukturanpassung (durch Symmetrieanpassung) noch groß genug für starke Bindung sein.

Die Zentralen Gruppen haben die Aufgaben,
a) die Symmetrie des Proteins auf den Hemmstoff zu übertragen,
b) die für eine gute Bindung mitverantwortlichen Seitenarme im richtgen Abstand und Bindungswinkel zu halten, und
c) selbst durch gute Einpassung in das Protein, z.B. das aktive Zentrum eines Enzyms, zur guten Bindung des Hemmstoffes und damit zur Hemmung des Proteins beizutragen.

Die zentralen Gruppen müssen selbst nicht unbedingt symmetrisch sein, z.B. wenn die Seitenarme weitgehend für die Affinität verantwortlich sind. Die Zentrale Gruppe kann chiral sein, z.B. Statin. Wichtig ist, daß die Vermittlung der Orientierung der Seitenarme und der richtigen Abstände optimal ist. Dies ist jedoch für den präparativen Chemiker in Kenntnis der Symmetrie oder eventuell sogar der genaueren Struktur des Enzyms kein Problem. Die Größe der Zentralen Gruppe kann verschieden sein, ebenso ihre chemische Natur, so daß auch anorganische Gruppen wie -P(O)OH-als zentrale Gruppe gelten kann. Ein Strukturmimikry des Substrats oder eines Übergangszustandes einer enzymatischen Reaktion durch den Hemmstoff kann wichtig sein.

Ungeeignet sind zentrale Gruppen, wenn sie zwar die richtigen Seitenketten (entsprechende Reihenfolge und Chiralität der Aminosäuren) im richtigen Abstand anordnen, aber so, daß ein Arm bezüglich der Richtung der Symmetrieachse falsch angeordnet ist, z.B. wenn "oben" und "unten" verkehrt sind. 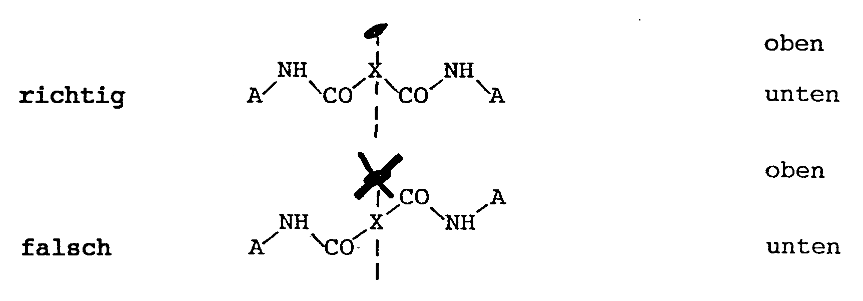
Die folgenden Beispiele zeigen teilweise oder annähernd symmetrische Peptide, die zu einer Hemmung der HI-Viren in H9-Zellen führen.

Bei den folgenden Beispielen bedeuten R und R' Peptidreste, vorzugsweise solche bis maximal 9 Aminosäuren, insbesondere mit 2 bis 4 Aminosäuren, oder andere kurze organisch-chemische Reste, beispielsweise CH₃(CH₂)ₙCO- mit n = 1 bis 10, CH₃CO-, H-, -NH₂, -NHR, -OR; X bedeutet kleine Aminosäurereste, wie Gly, Ala, Ser, oder andere kleine Reste, A und B sind Aminosäuren oder andere organische Reste. Dies gilt für alle folgenden Beispiele, wenn nichts anderes angegeben ist.

### BEISPIEL 1

Acetyl-(D)-Arg-(D)-Ala-(D)-Asn-Statin-(L)-Asn-(L)-Ala-(L)-Arg-NH₂
Acetyl-(L)-Arg-(L)-Ala-(L)-Gln-Statin-(D)-Gln-(D)-Ala-(D)-Arg-OH
Fluoracetyl-(L)-Arg-(L)-Ala-(L)-Asn-Statin-(D)-Asn-(D)-Ala-(D)-Arg-NH₂
Acetyl-(D)-Arg-(D)-Ala-(D)-Leu-Statin-(L)-Leu-(L)-Ala-(L)-Arg-NH₂
In den verwendeten Verbindungen kann ein Teil der Peptidkette aus Aminosäureresten einer räumlichen Konfiguration bestehen, während die andere Hälfte aus Aminosäuren der umgekehrten Konfiguration besteht, dergestalt, daß eine symmetrische oder annähernd symmetrische oder teilweise symmetrische Gesamtverbindung entsteht, z.B. entsprechend des in den Formeln
(L)-C-(L)-B-(L)-A-(D)-A-(D)-B-(D)-C
oder
(D)-C-(D)-B-(D)-A-(L)-A-(L)-B-(L)-C
ausgedrückten Prinzips, wobei A, B, C Aminosäurereste darstellen. Hierbei ist darauf zu achten, daß durch Einfügen einer geeigneten Zentralen Gruppe M (im Zentrum) das auf Seite 8 unten dargelegte Prinzip gewahrt ist.

Auch hier ist bei Hemmung für Protease die Enzymhemmung dadurch zu erreichen, daß die Enzymhemmer anstelle der spaltbaren Peptidbindungen eine nichtspaltbare Bindung besitzen, wie dies etwa bei Beispiel 2 erläutert ist, und daß in den verwendeten Verbindungen an eine zentrale organisch-chemische Gruppe mit zwei gleichen Substituenten zwei Peptide mit gleicher oder annähernd gleicher und sich entsprechender Aminosäuresequenz und gleicher Konfiguration bzw. Chiralität, aber mit umgekehrter Laufrichtung der Peptidbindungen so angeheftet werden, daß insgesamt eine räumlich-symmetrische oder annähernd symmetrische oder teilweise symmetrische Gesamtverbindung entsteht, wie dies auch für Beispiel 2 gezeigt ist,
ferner, daß wie auch in Beispiel 3 gezeigt, in den verwendeten Verbindungen an eine zentrale organisch-chemische Gruppe mit zwei unterschiedlichen Substituenten zwei Peptide mit gleicher oder annähernd gleicher und sich entsprechender Aminosäuresequenz mit gleicher Laufrichtung der Peptidbindungen, aber mit umgekehrter Chiralität der Aminosäuren so angeheftet werden, daß insgesamt eine räumlich-symmetrische oder annähernd oder teilweise symmetrische Gesamtverbindung entsteht,
und schließlich, daß in den verwendeten Verbindungen an eine symmetrische oder teilweise symmetrische oder annähernd symmetrische Verbindung chemisch reaktive Reste, z.B. entsprechend den Formeln XCH₂CO-, N₂CHCO-, NC-CH₂-CO-, RO₂C-, CH₂=CR-, ROₙS-, HS-, RO(H₂N=)C⁺- so angeheftet werden, daß die Verbindungen von Zielenzym reversibel oder irreversibel gebunden werden können. Hier bedeutet X Halogen, R ist ein Esterrest mit 1 bis 12 Kohlenstoffatomen, aber vorzugsweise ein C₁-C₃-Rest, oder ein Phenyl- oder Benzylrest und n = 1 bis 3.

### BEISPIEL 2

Acetyl-Arg-Leu-Asn-NH-(CH₂)₃-NH-Asn-Leu-Arg-Acetyl
Acetyl-Arg-Leu-Asn-NH-CH₂-O-CH₂-NH-Asn-Leu-Arg-Acetyl
Acetyl-Arg-Leu-Asn-NH-CH₂-CHOH-CH₂-NH-Asn-Leu-Arg-Acetyl
Bei den verwendeten Verbindungen wird im Falle von Proteinasen als Zielenzym die Enzymhemmung dadurch erreicht, daß sie anstelle der spaltbaren Peptidbindungen eine nichtspaltbare Bindung besitzen, z.B. nach den Formeln
-S-S-, -S-, -O-,
-CO-CHR-CH(OH)-CHR'-CO-, -NR-NR'-,
-NH-CHR-CH(OH)-CHR'-NH-, -NH-CF₂-CO-CH₂-NH-,
-NH-CF₂-CO-CF₂-NH-, -CO-(CH₂)₃-CO-,
-NH-(CH₂)₃-NH-, -CO-CH₂-O-CH₂-CO-, -N(OR)-, -NR-,
-P(O)ₙOH-, -CO-CHR-CO-,
-NH-CH₂-O-CH₂-NH-, -CO-CH₂-NR-CH₂-CO-,
-N(C₅H₁₁)-CF₂-CO-CF₂-N(C₅H₁₁)-,
-N(C₄H₉)-CH₂-CH(OH)-CH₂-N(C₄H₉)-,
-(2S,3S)-NH-CH(CH₂C₆H₁₁)-CH(OH)-CH₂-NR-,
oder ähnliche Verbindungen, wobei R und R' Wasserstoff oder Aryl- oder Alkylreste bis C₁₂ bedeuten und n die Zahl 1 oder 2 bedeutet.

In den verwendeten Verbindungen werden an eine zentrale organisch-chemische Gruppe mit zwei gleichen Substituenten zwei Peptide mit gleicher oder annähernd gleicher oder sich entsprechender Aminosäuresequenz und gleicher Konfiguration, aber mit umgekehrter Laufrichtung so angeheftet, daß insgesamt eine räumlich symmetrische oder annähernd symmetrische oder teilweise symmetrische Gesamtverbindung entsteht, z.B. entsprechend den Formeln
(L)-A-CONH-(L)-B-CONH-(L)-C-CONH-M-NHCO-(L)-C-NHCO-(L)-B-NHCO-(L)-A oder
(L)-C-NHCO-(L)-B-NHCO-(L)-A-NHCO-M-CONH-(L)-A-CONH-(L)-B-CONH-(L)-C oder
(L)-A-CONH-(D)-B-CONH-(L)-C-CONH-M-NHCO-(L)-C-NHCO-(L)-B-NHCO-(L)-A oder
(L)-C-NHCO-(L)-B-NHCO-(L)-A-NHCO-M-CONH-(L)-A-CONH-(L)-B-CONH-(D)-C oder
(L)-B-CONH-(L)-B-CONH-(L)-C-CONH-M-NHCO-(L)-C-NHCO-(L)-B-NHCO-(L)-A oder
(L)-B-NHCO-(L)-A-NHCO-M-CONH-(L)-A-CONH-(L)-B-CONH-(L)-C,
wobei A, B, C Aminosäurereste und M eine zentrale organisch-chemische Gruppe (Beispiele s.o.) darstellen.

### BEISPIEL 3

Zusätzlich zu den in oben in Beispiel 7 gezeigten Möglichkeiten werden hier in den verwendeten Verbindungen an eine zentrale organisch-chemische Gruppe mit zwei unterschiedlichen Substituenten zwei Peptide mit gleicher oder annähernd gleicher oder sich entsprechender Aminosäuresequenz, aber mit umgekehrter Laufrichtung und Konfiguration der Aminosäuren, aber gleicher Laufrichtung der Peptidbindungen, so angeheftet, daß insgesamt eine räumlich symmetrische oder annähernd symmetrische oder teilweise symmetrische Gesamtverbindung entsteht, z.B. entsprechend den Formeln
(L)-A-CONH-(L)-B-CONH-(L)-C-CONH-M-CONH-(D)-C-CONH-(D)-B-CONH-(D)-A oder
(L)-A-CONH-(D)-B-CONH-(L)-C-CONH-M-CONH-(D)-C-CONH-(D)-B-CONH-(D)-A oder
(D)-A-CONH-(D)-B-CONH-(D)-C-CONH-M-CONH-(L)-C-CONH-(L)-B-CONH-(L)-A, oder
(D)-A-NHCO-(D)-B-NHCO-(D)-C-NHCO-M-NHCO-(L)-C-NHCO-(L)-B-NHCO-(D)-A
wobei A, B, C Aminosäurereste und M eine zentrale Gruppe darstellen.

Bei den verwendeten Verbindungen wird im Falle von Proteinasen als Zielenzym die Enzymhemmung dadurch erreicht, daß sie anstelle der spaltbaren Peptidbindungen eine nichtspaltbare Bindung besitzen, z.B. nach den Formeln
-CR₂-NH-, -CH(OH)-NH-, -CO-N(CH₃)-, -P(O)ₙ-NH-,
-(3S,4S)-4-Amino-3-hydroxy-6-methylheptansäure- (Statin),
-(3S,4S)-3-Hydroxy-4-amino-5-phenylpentansäure (AHPPA),
oder ähnliche Verbindungen, wobei R und R' Wasserstoff oder Aryl- oder Alkylreste bis C₁₂ bedeuten und n die Zahl 1 oder 2 bedeutet.

### BEISPIEL 4

NH₂-Arg-Leu-Asn-CO-(CH₂)₃-CO-Asn-Leu-Lys-NH₂
H₂N-(D)-Leu-(D)-Asn-CO-(CH₂)₃-CO-(D)-Asn-(D)-Ile-NH₂
NH₂-Arg-Leu-Asn-CO-CH₂-CHOH-CH₂-CO-Asn-Leu-Arg-NH₂
Acetyl-Arg-Leu-Asn-NH-CH₂-O-CH₂-NH-Asn-Leu-Arg-Acetyl
Acetyl-Arg-Leu-Asn-NH-CH₂-CH(OH)-CH₂-NH- Asn-Leu-H
H-Ala-Ala-Statin-(D)-Val-(D)-Val-OCH₃
Zusätzlich zu den in den beiden vorhergehenden Beispielen angegebenen Möglichkeiten können hier in den verwendeten Verbindungen an eine zentrale organisch-chemische Gruppe zwei Peptide mit unterschiedlichen Aminosäuresequenzen, aber mit ähnlicher bzw. entsprechender Verteilung von Resten mit gleicher elektrischer Ladung oder gleicher oder ähnlicher Hydrophobizität oder Hydrophilität oder Größe der Seitenketten oder einer anderen physikalisch-chemischen Eigenschaft so eingebaut werden, daß insgesamt eine räumlich annähernd symmetrische oder teilweise symmetrische Gesamtverbindung entsteht, z.B. entsprechend den Formeln
(L)-C-(L)-A⁺-CONH-M-CONH-(D)-B⁺-(D)-C, oder
(L)-C-(L)-A⁺-CONH-M-NHCO-(L)-B⁺-(L)-C, oder
(L)-C-(L)-A⁺-CONH-M-CONH-(D)-B⁺-(D)-D, oder
(L)-C-(L)-A⁺-CONH-M-CONH-(D)-B⁺-(L)-C, oder
(L)-D-(L)-AX-CONH-M-NHCO-(L)-BX-(L)-C, oder
(L)-C-(L)-AX-CONH-M-CONH-(D)-BX-(D)-C, oder
wobei A⁺, B⁺ = zwei verschiedene Aminosäurereste mit gleicher Ladung, M eine zentrale organisch-chemische Gruppe und AX, BX zwei verschiedene Aminosäuren mit vergleichbar großen hydrophoben oder hydrophilen Seitenketten sind.

Abschließend seien noch einige Beispiele für zentrale Gruppen (M), für Seitenketten sowie für ganze Inhibitoren gezeigt:
Beispiele für Zentrale Gruppen:
-NH-CH(OH)-CH(OH)-NH-, -O-, Statin,
-NH-CH(CH₂C₆H₁₁)-CH(OH)-CH₂-NH-,
-NH-CH(C₄H₉)-CO-CH(C₄H₉)-NH-,
-NH-CH₂-CH(OH)-CH₂-NH-,
(1S,3S)-NH-CH(Cyclohexylmethyl)-CO-CH(Cyclohexylmethyl)-NH-, 2-Alkylstatin, -CH₂-, Ethylenepoxid, Thiophen,
Beispiele für Seitenketten:
Ac-Ser-Gln-Asn-Tyr-, H-His-Pro-His-Tyr-,
Ac-Arg-Ser-Gln-His-Cha-, H-Ala-Ala-
Beispiele für ganze Inhibitoren:
H-His-Pro-His-NH-CHR-CH(OH)-CH₂-NH-His-Pro-His-H
(R=-CH₂-C₆H₁₁ etc.)
Ac-Arg-Ser-Gln-Asn-
-NH-CH(CH₂C₆H₁₁)-CO-CH(CH₂C₆H₁₁)-NH-
-Asn-Gln-Ser-Arg-Ac
(Zentrale Gruppe: 1S,3S; statt CO auch -CH(OH)-, -CO-CO-, -CH(OH)-CH(OH)-, Furan, Ethylenepoxid etc.)
tBoc-His-Pro-Phe-His-Leu-Statin-D-His-D-Phe-D-Pro-D-His- tBoc
Ein Verfahren zur Inhibierung von Proteasen, z.B. HIV-Protease durch bestimmte Peptide besteht, kurz skizziert, darin, daß man
1. die Symmetrie des zu hemmenden Enzyms bestimmt,
2. die Sequenz eines guten Substrats oder eines hemmenden Peptids auswählt und
3. die dem Symmetriezentrum am nächsten liegenden Aminosäuren feststellt,
4. ein solches, die Bindung vermittelndes Peptid an diesen Aminosäuren durch chemische Synthese so mittels einer zentralen Gruppe verknüpft, daß ein hinreichend symmetrisches Peptid entsteht,
5. wobei man eine solche zentrale Gruppe wählt, daß die sich entsprechenden Aminosäuren im richtigen Abstand zu Mitte stehen,
6. mittels Computer aided molecular design die gute Paßform überprüft und die genaue Einhaltung der Symmetrie im Inhibitor feststellt und
7. die Hemmaktivität überprüft, insbesondere wenn die Strukturkoordinaten nicht bekannt sind. In diesem Fall wird die Sequenz der Seitenarme und die Struktur der zentralen Gruppe durch trial and error über die Hemmaktivität optimiert.

Die chemische Herstellung solcher Verbindungen ist an sich bekannt, ebenso ist die Verabreichung der Verbindungen an sich bekannt, so daß der Fachmann hier auf übliche und wohlbekannte Arbeitsweisen zurückgreifen kann.

Abschließend seien noch einige bevorzugte Ausführungsformen hinsichtlich Hemmverbindungen angegeben:
Die verwendeten Verbindungen bestehen vorzugsweise aus Peptiden oder Derivaten davon, wobei z.B.
folgende symmetrische oder teilweise symmetrische Verbindungen in betracht gezogen werden:
X-Y-Z-M-Z-Y-X, Z-M-Z, R-U-X-Y-Z-M-Z, wobei X,Y,Z,U,R Aminosäuren oder Derivate davon sind, M die zentrale organisch-chemische Gruppe darstellt und die beiden Strukturen Y, die zu beiden Seiten der zentralen Gruppe stehen, strukturell oder in ihren physikalisch-chemischen Eigenschaften ähnliche Verbindungen sind, was zusätzlich oder stattdessen auch für die anderen genannten Gruppen X, Z, U oder R gelten kann. Bei guter Passung kann eine symmetrische oder fast symmetrische Gruppe M zur Hemmung ausreichen, z.B. ein Dipeptidanalogon, wie es auf Seite 20, Zeile 18-20 für die Gruppe M von -NH-... bis ...-NH- gezeigt ist.
Die Voraussetzung zur Bindung der verwendeten Verbindungen an die HIV-Protease kann z.B. dadurch geschaffen werden, daß in ihnen typische Spaltsequenzen oder Bindungssequenzen der natürlichen Substrate oder mit ihnen verwandten Strukturen oder Strukturen dieser Art verwendet werden, die so modifiziert wurden, daß sie dem Zielenzym nicht mehr als Substrat dienen und als Inhibitoren wirken. In den verwendeten Verbindungen können die Voraussetzungen zur Hemmung der Zielenzyme auch dadurch erreicht werden, daß Substrate oder substratähnliche Verbindungen so modifiziert werden, daß sie anstelle der enzymatisch veränderbaren Stellen nicht mehr veränderbare Stellen tragen und daher als Inhibitoren wirken.

Eine bevorzugte Gruppe von Hemmverbindungen im Falle von Proteinasen als Zielenzym haben anstelle der spaltbaren Peptidbindungen ein Dipeptidanalogon mit reduzierter Peptidbindung oder eine andere ähnliche Verbindung mit nichtspaltbarer Bindung und gleicher oder ähnlicher Länge wie ein Dipeptid und wirken so für das Zielenzym als Inhibitoren. Die verwendeten Verbindungen können im Falle von Proteinasen als Zielenzym anstelle der spaltbaren Peptidbindung Statin oder ein Derivat des Statins besitzen. Sie können aber auch im Falle von Proteinasen als Zielenzym anstelle der spaltbaren Peptidbindungen eine phosphorhaltige nichtspaltbare Verbindung mit gleicher oder ähnlicher Länge wie ein Dipeptid besitzen, wie z.B. Phosphonsäureamide. Statin und ähnliche Dipeptidanaloga stellen selbst bereits annähernd symmetrische Verbindungen dar (Symmetrie nahe am -OH).

Bei Proteinasen als Zielenzym kann in den Hemmverbindungen durch Einführung einer längeren Aminosäure oder einer anderen organisch-chemischen Gruppe die Stelle der spaltbaren Peptidbindung im Vergleich zu der Lage der Spaltstelle in einem guten Substrat räumlich verschoben werden, wodurch die Verbindung für das Zielenzym als Inhibitor wirkt. Es kann z.B. in den verwendeten Verbindungen die Stelle der spaltbaren Peptidbindungen durch Einführung von Statin oder einer anderen Gruppe im Vergleich zu der Bindungslage eines guten Substrats räumlich verschoben werden.
In den verwendeten Verbindungen kann die Symmetrie oder teilweise Symmetrie der Verbindungen dadurch erreicht werden, daß in den Verbindungen zentrale organisch-chemische Gruppen vorhanden sind, die als Zentren der Symmetrie oder der annähernden Symmetrie wirken, oder die verwendeten Verbindungen besitzen zentrale organisch-chemische Gruppen mit zwei identischen oder in ihrer Funktion äquivalenten organischen Substituenten, die mit zwei identischen oder teilweise identischen Peptiden oder peptidähnlichen Verbindungen so reagieren können, daß eine symmetrische oder teilweise symmetrische Gesamtverbindung entsteht, z.B. entsprechend den Formeln
C-CONH-B-CONH-A-CONH-M-NHCO-A-NHCO-B-NHCO-C
oder
C-NHCO-B-CHCO-A-NHCO-M-CONH-A-CONH-B-CONH-C,
wobei A, B, C Aminosäurereste und M eine zentrale organisch-chemische Gruppe darstellen. Die verwendeten Verbindungen können eine symmetrische oder annähernd symmetrische zentrale organisch-chemische Gruppe mit zwei identischen oder in ihrer Funktion äquivalenten organischen Substituenten besitzen, die in der Länge mindestens einem Dipeptid entsprechen und mit Peptiden oder so reagieren können, daß eine symmetrische oder annähernd symmetrische oder teilweise symmetrische Gesamtverbindung entsteht. Falls das oben erstgenannte Formelbeispiel (...-NH-M-NH-...) einem guten Inhibitor entspricht, muß im zweiten Beispiel (...-CO-M-CO-...) die Chiralität der verwendeten gleichen Aminosäuren (A,B,C) umgedreht werden ("D"-Formen), um eine ähnlich gute Passung und damit Hemmung zu erreichen.

Für den Fall einer zentralen organisch-chemischen Gruppe mit zwei ungleichen Substituenten, an die zwei gleiche oder annähernd gleiche oder sich entsprechende Peptide oder peptidähnliche Verbindungen angeheftet sind, seien als Beispiele die Formeln
C-CONH-B-CONH-A-CONH-M-CONH-A-CONH-B-CONH-C
oder
C-NHCO-B-NHCO-A-NHCO-M-NHCO-A-NHCO-B-NHCO-C
genannt, wobei A, B, C Aminosäurereste und M die zentrale organisch-chemische Gruppe sind. Die symmetrische Sequenzanordnung allein reicht im allgemeinen nicht aus für die Konstitution eines hinreichend "symmetrischen" Inhibitors, wie nachstehend noch erläutert wird (s.a. Seite 8).

In den verwendeten Verbindungen kann an ein Peptid oder an eine peptidähnliche Verbindung ein Nichtpeptidrest so gebunden werden, daß eine annähernd symmetrische oder teilweise symmetrische Gesamtverbindung entsteht, z.B. in bezug auf eine oder mehrere physikalisch-chemische Eigenschaften wie Ladung, Hydrophilizität, Hydrophobizität oder Größe der Seitenkette bzw. des Restes, oder in den verwendeten Verbindungen ist an ein Peptid oder an eine peptidähnliche Verbindung oder an ein Peptid mit einer zentralen organisch-chemischen Gruppe eine Seitenkette oder ein Nichtpeptidrest so gebunden, z.B. entsprechend den Formeln B-A-M-A-R oder R-B-A-M-A oder B-A-M-R,
wobei A,B,C,D, Aminosäurereste, M eine zentrale organisch-chemische Gruppe und R einen organischen Rest darstellt, dergestalt, daß insgesamt eine räumlich annähernd symmetrische oder teilweise symmetrische Gesamtverbindung entsteht, z.B. in bezug auf eine oder mehrere physikalisch-chemische Eigenschaften, wie Ladung, Hydrophiliziät, Hydrophobizität oder Größe der Seitenkette bzw. des Restes.
Es können aber an ein symmetrisches oder teilweise symmetrisches oder annähernd symmetrisches Peptid oder eine peptidähnliche Verbindung chemisch reaktive Reste, z.B. entsprechend den Formeln XCH₂CO-, N₂CHCO-, NC-CH₂-CO-, RO₂C-, CH₂=CR-, ROₙS-, HS-, RO(H₂N=)C⁺-, so gebunden sein, daß die Verbindungen vom Zielenzym reversibel oder irreversibel gebunden werden können. Auch hier bedeutet n die zahl 1 oder 2 und R ist ein üblicher Esterrest, wie schon früher angegeben.
Die verwendeten Verbindungen können auch Aminosäuresequenzen der Enzyme oder Proteine enthalten, die für die Assoziation ihrer Untereinheiten oder Teilstrukturen oder die Stabilität oder strukturelle Anordnung der HIV-Protease mitverantwortlich sind, oder verwandte oder ähnliche Aminosäuresequenzen oder strukturell ähnliche organisch-chemische Reste besitzen, so daß die Verbindungen die Struktur oder Stabilität der HIV-Protease beeinträchtigen oder ihre enzymatische Aktivität oder ihre Funktion beeinträchtigen oder ihre Bildung verändern können. Hierbei können die verwendeten Verbindungen Aminosäuresequenzen oder verwandte oder ähnliche Aminosäuresequenzen oder strukturell ähnliche organisch-chemische Reste der HIV-Protease enthalten, die für die Assoziation der Untereinheiten des Enzyms und die Bildung oder den Zusammenhalt des funktionierenden Enzymkomplexes mitverantwortlich sind, so daß die Verbindungen die Struktur oder Stabilität der Enzymkomplexe beeinträchtigen oder ihre enzymatische Aktivität verringern oder ihre Bildung verhindern können.
Die verwendeten Verbindungen können vorzugsweise die Aminosäuresequenz Trp-Lys-Pro-Lys-Met-Ile-Gly-Gly-Ile-Gly-Gly-Phe-Ile-Lys-Val-Arg; Gln-Ile-Leu-Ile-Glu-Cys; Val-Gly-Pro-Thr-Pro-Val-Asn; Ile-Gly-Arg-Asn; Ala-Gly-Arg-Asn-Leu-Leu-Thr-Gln-Ile oder verwandte oder ähnliche Aminosäuresequenzen oder strukturell ähnliche organisch-chemische Reste oder Teile daraus enthalten, die für die Assoziation der Untereinheiten der HIV-Protease und die Bildung oder den Zusammenhalt des funktionierenden Enzymkomplexes mitverantwortlich sind, so daß die Verbindungen die Struktur oder Stabilität der Enzymkomplexe beeinträchtigen oder ihre enzymatische Aktivität verringern oder ihre Bildung verhindern können.

Auf diese Weise kann also Struktur und/oder die Wirkung von HIV-Proteasen, die eine gewisse Symmetrie haben, indem sie aus gleichen oder ungleichen Untereinheiten bestehen, durch stabile organisch-chemische Verbindungen gehemmt werden, was zur Therapie dienen kann, wenn die verwendeten Verbindungen Aminosäuresequenzen der strukturell unsymmetrischen komplexen Zielenzyme oder verwandte oder ähnliche Aminosäuresequenzen oder strukturell ähnliche organisch-chemische Reste enthalten, die für die Assoziation der Untereinheiten der Enzyme und die Bildung und den Zusammenhang der funktionierenden Enzymkomplexe mitverantwortlich sind, so daß die Verbindungen die Bildung oder den Zusammenhalt oder die Stabilität der Enzymkomplexe stören und ihre Aktivität beeinträchtigen oder verhindern können.

## Patentansprüche

1. HIV-Proteaseinhibitor mit einer zentralen Gruppe M und zwei Peptid-Seitenarmen, dadurch gekennzeichnet, daß M aus der Gruppe von
-S-S-, -S-, -O-,
-CO-CHR-CH(OH)-CHR'-CO-, -NR-NR'-,
-NH-CHR-CH(OH)-CHR'-NH-, -NH-CF₂-CO-CH₂-NH-,
-NH-CF₂-CO-CF₂-NH-, -CO-(CH₂)₃-CO-,
-NH-(CH₂)₃-NH-, -CO-CH₂-O-CH₂-CO-, -N(OR)-, -NR-,
-P(O)ₙOH-, -CO-CHR-CO-,
-NH-CH₂-O-CH₂-NH-, -CO-CH₂-NR-CH₂-CO-,
-N(C₅H₁₁)-CF₂-CO-CF₂-N(C₅H₁₁)-,
-N(C₄H₉)-CH₂-CH(OH)-CH₂-N(C₄H₉)-,
-(2S,3S)-NH-CH(CH₂C₆H₁₁)-CH(OH)-CH₂-NR-,
-CR₂-NH-, -CH(OH)-NH-, -CO-N(CH₃)-, -P(O)ₙ-NH-,
-(3S,4S)-4-Amino-3-hydroxy-6-methylheptansäure (Statin),
-(3S,4S)-3-Hydroxy-4-amino-5-phenylpentansäure (AHPPA),
-NH-CH(OH)-CH(OH)-NH-,
-NH-CH(CH₂C₆H₁₁)-CH(OH)-CH₂-NH-,
-NH-CH(C₄H₉)-CO-CH(C₄H₉)-NH-,
-NH-CH₂-CH(OH)-CH₂-NH-,
(1S,3S)-NH-CH(Cyclohexylmethyl)-CO-CH(Cyclohexylmethyl)-NH-, 2-Alkylstatin, -CH₂-, Ethylenepoxid, Thiophen,
wobei R und R' Wasserstoff oder Aryl- oder Alkylreste bis C₁₂ bedeuten und n die zahl 1 oder 2 bedeutet,
ausgewählt ist, die Peptid-Seitenarme Aminosäuren und/oder Aminosäurederivate enthalten, die gleich oder annähernd gleich und in bezug auf die zentrale Gruppe M symmetrisch oder annähernd symmetrisch sind,und der Inhibitor zumindest bezüglich des hemmbaren Teils der HIV-Protease eine lokale Symmetrie aufweist.

2. HIV-Proteaseinhibitor mit einer zentralen Gruppe M und den Peptid-Seitenarmen Ac-Arg-Ser-Gln-Asn und-Asn-Gln-Ser-Arg-Ac, dadurch gekennzeichnet, daß die zentrale Gruppe M aus
-NH-CH(CH₂C₆H₁₁)-CO-CH(CH₂C₆H₁₁)-NH-,
-NH-CH(CH₂C₆H₁₁)-CO-CO-CH(CH₂C₆H₁₁)-NH-,
-NH-CH(CH₂C₆H₁₁)-CH(OH)-CH(OH)-CH(CH₂C₆H₁₁)-NH-,
-NH-CH(CH₂C₆H₁₁)-Furan-CH(CH₂C₆H₁₁)-NH-,
-NH-CH(CH₂C₆H₁₁)-Ethylenepoxid-CH(CH₂C₆H₁₁)-NH-,
ausgewählt ist.

## Claims

1. A HIV protease inhibitor having a central group M and two peptide side chains, characterized in that M is selected from the group consisting of
-S-S-, -S-, -O-,
-CO-CHR-CH(OH)-CHR'-CO-, -NR-NR'-,
-NH-CHR-CH(OH)-CHR'-NH-, -NH-CF₂-CO-CH₂-NH-,
-NH-CF₂-CO-CF₂-NH-, -CO-(CH₂)₃-CO-,
-NH-(CH₂)₃-NH-, -CO-CH₂-O-CH₂-CO-, -N(OR)-, -NR-,
-P(O)ₙOH-, -CO-CHR-CO-,
-NH-CH₂-O-CH₂-NH-, -CO-CH₂-NR-CH₂-CO-,
-N(C₅H₁₁)-CF₂-CO-CF₂-N(C₅H₁₁)-,
-N(C₄H₉)-CH₂-CH(OH)-CH₂-N(C₄H₉)-,
-(2S,3S)-NH-CH(CH₂C₆H₁₁)-CH(OH)-CH₂-NR-,
-CR₂-NH-, -CH(OH)-NH-, -CO-N(CH₃)-, -P(O)ₙ-NH-,
-(3S,4S)-4-amino-3-hydroxy-6-methylheptanoic acid (statin),
-(3S,4S)-3-hydroxy-4-amino-5-phenylpentanoic acid (AHPPA),
-NH-CH(OH)-CH(OH)-NH-,
-NH-CH(CH₂C₆H₁₁)-CH(OH)-CH₂-NH-,
-NH-CH(C₄H₉)-CO-CH(C₄H₉)-NH-,
-NH-CH₂-CH(OH)-CH₂-NH-,
(1S,3S)-NH-CH(cyclohexylmethyl)-CO-CH(cyclohexylmethyl)-NH-, 2-alkyl statin, -CH₂-, ethylene epoxide, thiophene,
wherein R and R' represent hydrogen or aryl or alkyl residues up to C₁₂ and n is 1 or 2,
the peptide side chains contain amino acids and/or amino acid derivatives which are equal or approximately equal and symmetric or approximately symmetric in relation to the central group M, and the inhibitor shows local symmetry at least with respect to the HIV protease portion which can be inhibited.

2. The HIV protease inhibitor having a central group M and the peptide side chains Ac-Arg-Ser-Gln-Asn and -Asn-Gln-Ser-Arg-Ac, characterized in that the central group M is selected from
-NH-CH(CH₂C₆H₁₁)-CO-CH(CH₂C₆H₁₁)-NH-,
-NH-CH(CH₂C₆H₁₁)-CO-CO-CH(CH₂C₆H₁₁)-NH-,
-NH-CH(CH₂C₆H₁₁)-CH(OH)-CH(OH)-CH(CH₂C₆H₁₁)-NH-,
-NH-CH(CH₂C₆H₁₁)-furan-CH(CH₂C₆H₁₁)-NH-,
-NH-CH(CH₂C₆H₁₁)-ethylene epoxide-CH(CH₂C₆H₁₁)-NH-.

## Revendications

1. Inhibiteur de la protéase-HIV comportant un groupe central M et deux chaînes latérales, caractérisé en ce que M est choisi dans le groupe comprenant :
-S-S-, -S-, -O-,
-CO-CHR-CH(OH)-CHR'-CO-, -NR-NR'-,
-NH-CHR-CH(OH)-CHR'-NH-, -NH-CF₂-CO-CH₂-NH-,
-NH-CF₂-CO-CF₂-NH-, -CO-(CH₂)₃-CO-,
-NH-(CH₂)₃-NH-, -CO-CH₂-O-CH₂-CO-, -N(OR)-, -NR-
-P(O)ₙOH-, -CO-CHR-CO-,
-NH-CH₂-O-CH₂-NH-, -CO-CH₂-NR-CH₂-CO-,
-N(C₅H₁₁)-CF₂-CO-CF₂-N(C₅H₁₁)-,
-N(C₄H₉)-CH₂-CH(OH)-CH₂-N(C₄H₉)-,
-(2S, 3S)-NH-CH(CH₂C₆H₁₁)-CH(OH)-CH₂-NR-,
-CR₂-NH-, -CH(OH)-NH-, -CO-N(CH₃)-, -P(O)ₙ-NH-,
-acide-(3S,4S)-4-amino-3-hydroxy-6-méthyl-heptanoïque (Statine)
-acide-(3S,4S)-3-hydroxy-4-amino-5-phényl- pentanoïque (AHPPA),
-NH-CH(OH)-CH(OH)-NH-,
-NH-CH(CH₂C₆H₁₁)-CH(OH)-CH₂-NH-,
-NH-CH(C₄H₉)-CO-CH(C₄H₉)-NH-,
-NH-CH₂-CH(OH)-CH₂-NH-,
(1S,3S)-NH-CH(cyclohexylméthyl)-CO-CH(cyclohexylméthyl)-NH-, 2-alkylstatine, -CH₂-, éthylène-époxyde, thiophène,
où R et R' indiquent l'hydrogène ou un aryle ou un reste d'aryle jusqu'en C₁₂ et n signifie 1 ou 2, les chaînes latérales peptidiques contiennent des aminoacides et/ou des dérivés d'aminoacides, qui sont totalement ou approximativement symétriques par rapport au groupe central M, et l'inhibiteur présente une symétrie locale au moins au niveau de la partie de la protéase HIV qui est susceptible d'inhibition.

2. Inhibiteur de la protéase HIV avec un groupe central M et les chaînes latérales peptidiques Ac-Arg-Ser-Gln-Asn et Asn-Gln-Ser-Arg-Ac, caractérisé en ce que le groupe central est choisi parmi
-NH-CH(CH₂C₆H₁₁)-CO-CH(CH₂C₆H₁₁)-NH-,
-NH-CH(CH₂C₆H₁₁)-CO-CO-CH(CH₂C₆H₁₁)-NH-,
-NH-CH(CH₂C₆H₁₁)-CH(OH)-CH(OH)-CH(CH₂C₆H₁₁)-NH-,
-NH-CH(CH₂C₆H₁₁)-Furanne-CH(CH₂C₆H₁₁)-NH-,
-NH-CH(CH₂C₆H₁₁)-éthylène-époxyde-CH(CH₂C₆H₁₁)-NH-
